# EUROPÄISCHE PATENTSCHRIFT

(11) **EP 0 297 354 B1**
(45) Veröffentlichungstag und Bekanntmachung des Hinweises auf die Patenterteilung: **01.09.1993**
(21) Anmeldenummer: 88109562.4
(22) Anmeldetag: 15.06.1988
(51) Int. Cl.: A61B 17/34, A61B 10/00

(54) **Biopsieeinrichtung für ein Röntgenuntersuchungsgerät**
Biopsy device for an X-ray apparatus
Dispositif de biopsie pour un appareil diagnostique à rayon-X

(30) Priorität: 30.06.1987 DE 3721589
(43) Veröffentlichungstag der Anmeldung: 04.01.1989
(73) Patentinhaber: SIEMENS AKTIENGESELLSCHAFT, 80333 München (DE)
(72) Erfinder: Saffer, Edmund, D-8551 Eggolsheim (DE)

(56) Entgegenhaltungen:
- EP-A- 0 146 511
- FR-A- 2 521 000
- FR-A- 2 584 601
- US-A- 4 485 815

## Beschreibung

Die Erfindung betrifft eine Biopsieeinrichtung für ein Röntgenuntersuchungsgerät mit einem Röntgenstrahler mit einem Fokus, von dem eine Strahlenpyramide ausgeht, und einem diesem gegenüberliegend angeordneten Strahlungsempfänger zur Erzeugung eines Röntgenbildes eines zwischen beiden befindlichen Untersuchungsobjektes, welche Biopsieeinrichtung Mittel zur Bestimmung eines zu einem auf dem Röntgenbild abgebildeten, innerhalb des Untersuchungsobjektes befindlichen diagnostisch relevanten Objekt gehörigen Einstichpunktes für eine Biopsienadel aufweist und außerdem ein Führungselement zum Ausrichten der Biopsienadel auf den jeweiligen Einstichpunkt und Mittel zum Positionieren des Führungselementes relativ zu dem Untersuchungsobjekt aufweist.

Eine Biopsieeinrichtung zur Verwendung in der Mammographie ist aus der Werbedruckschrift der Fa. Siemens "Der moderne Mammographie-Arbeitsplatz: MAMMOMAT B", Best.-Nr.: A19100-M1087- A789-01, bekannt. Die bekannte Biopsieeinrichtung weist zwei einen Winkel einschließende, auf dem Röntgenbild abbildbare Maßstäbe auf, die an einer zur Kompression des Untersuchungsobjektes dienenden Platte angebracht sind. Die Maßstäbe sind an dem Rand einer in der Platte vorgesehenen Öffnung angebracht, durch die das Untersuchungsobjekt zugänglich ist. Außerdem sind Mittel vorgesehen, die es gestatten, ein Schattenkreuz, dessen Schenkel parallel zu den Maßstäben verlaufen, auf das Untersuchungsobjekt zu projizieren. Dabei weisen die Schenkel des Schattenkreuzes eine solche Länge auf, daß sie die Maßstäbe schneiden. Das Schattenkreuz ist derart verstellbar, daß die Lage des Kreuzungspunktes seiner Schenkel eine beliebige Position auf dem durch die Öffnung der Platte zugänglichen Bereich des Untersuchungsobjektes einnehmen kann.

Wird ein Röntgenbild von dem Untersuchungsobjekt angefertigt, werden die Maßstäbe zusammen mit diesem abgebildet. Zeigt das Röntgenbild ein diagnostisch relevantes Objekt innerhalb des Untersuchungsobjektes, das punktiert oder für eine Probeexzision markiert werden soll, werden auf dem Röntgenbild anhand der Maßstäbe die Koordinaten des Objektes bestimmt. Anschliessend wird das Schattenkreuz zur Bestimmung des Einstichpunktes für die Biopsienadel auf die entsprechenden Koordinaten eingestellt. Zur Punktierung bzw. Markierung wird dann mit der Biopsienadel am Kreuzungspunkt der Schenkel des Schattenkreuzes eingestochen und die Biopsienadel in die erforderliche Tiefe vorgetrieben. Der so ermittelte Einstichpunkt liegt auf einer Geraden, die durch das diagnostisch relevante Objekt und den Fokus des Röntgenstrahlers verläuft. Es versteht sich, daß das Untersuchungsobjekt in dem Zeitraum zwischen der Anfertigung des Röntgenbildes und der Vornahme der Punktion nicht bewegt werden darf.

Die bekannte Biopsieeinrichtung ist zwar sehr einfach aufgebaut und gestattet es, den Einstichpunkt für die Biopsienadel anhand eines einzigen Röntgenbildes sehr genau zu bestimmen, jedoch besteht besonders dann, wenn sich das diagnostisch relevante Objekt weit im Inneren des Untersuchungsobjektes befindet, die Gefahr, daß die Biopsienadel an dem Objekt vorbeigeführt wird. Falls dies nicht bemerkt wird, besteht die Gefahr von Fehldiagnosen. Zumindest muß jedoch die Punktion wiederholt werden, was für das Untersuchungsobjekt - einen Patienten - höchst unangenehm ist.

Dieser Nachteil ist im Falle einer in der EP-A-0 146 511 beschriebenen Biopsieeinrichtung der eingangs genannten Art vermieden. Diese Biopsieeinrichtung besitzt Mittel zur Bestimmung eines zu einem diagnostisch relevanten Objekt gehörigen Einstichpunktes für die Biopsienadel, die anhand zweier unter unterschiedlichen Winkeln aufgenommener Röntgenbilder, auf denen außer dem diagnostisch relevanten Objekt jeweils eine ortsfeste Marke abgebildet ist, durch einen Rechenvorgang einen Einstichpunkt für die Biopsienadel ermitteln. Dieser Einstichpunkt liegt auf einer Geraden, die durch das diagnostisch relevante Objekt und senkrecht zu der Oberfläche einer ebenen Unterlage für das Untersuchungsobjekt verläuft. Außerdem besitzt die bekannte Biopsieeinrichtung eine Führungseinrichtung für die Biopsienadel mit einem Kanal, dessen Längsachse rechtwinklig zu der Oberfläche der Unterlage für das Untersuchungsobjekt verläuft. Weiter sind Mittel zum Positionieren der Führungseinrichtung vorhanden, mittels derer die Führungseinrichtung in Richtung der drei Raumachsen verstellbar ist. Zur Durchführung einer Punktion wird die Führungseinrichtung derart positioniert, daß die Längsachse ihres Kanales durch den ermittelten Einstichpunkt und damit das diagnostisch relevante Objekt verläuft. Es ist somit zwar ausgeschlossen, daß die Biopsienadel an dem diagnostisch relevanten Objekt vorbeigeführt wird, jedoch sind zur Bestimmung des Einstichpunktes für die Biopsienadel zwei Röntgenbilder erforderlich, was zu einer unerwünscht hohen Strahlenbelastung des Untersuchungsobjektes führt. Außerdem ist die bekannte Röntgeneinrichtung kompliziert aufgebaut.

Der Erfindung liegt die Aufgabe zugrunde, eine Biopsieeinrichtung der eingangs genannten Art so auszubilden, daß es möglich ist, den Einstichpunkt anhand eines einzigen Röntgenbildes zu bestimmen, und es dennoch ausgeschlossen ist, daß die Biopsienadel an dem diagnostisch relevanten Objekt vorbeigeführt wird.

Diese Aufgabe wird nach der Erfindung gelöst durch eine Biopsieeinrichtung für ein Röntgenuntersuchungsgerät mit einem Röntgenstrahler mit einem Fokus, von dem eine Strahlenpyramide ausgeht, und einem diesem gegenüberliegend angeordneten Strahlungsempfänger zur Erzeugung eines Röntgenbilders eines zwischen beiden befindlichen Untersuchungsobjektes, welche Biopsieeinrichtung Mittel zur Bestimmung eines zu einem auf dem Röntgenbild abgebildeten, innerhalb des Untersuchungsobjektes befindlichen diagnostisch relevanten Objekt gehörigen Einstichpunktes für eine Biopsienadel aufweist, der auf einer Geraden liegt, die durch das diagnostisch relevante Objekt und den Fokus des Röntgenstrahlers verläuft und außerdem eine Führungseinrichtung zum Ausrichten der Biopsienadel auf den jeweiligen Einstichpunkt und Mittel zum Positionieren der Führungseinrichtung relativ zu dem Untersuchungsobjekt aufweist, mittels derer die Führungseinrichtung selbsttätig derart positionierbar ist, daß der Einstichkanal der in der Führungseinrichtung aufgenommenen Biopsienadel für beliebige Positionen der Führungseinrichtung relativ zu dem Untersuchungsobjekt auf der Geraden durch den Fokus des Röntgenstrahlers und das diagnostisch relevante Objekt liegt. Da die Mittel zum Bestimmen des Einstichpunktes derart ausgebildet sind, daß der jeweils bestimmte Einstichpunkt auf einer Geraden liegt, die durch das diagnostisch relevante Objekt und den Fokus des Röntgenstrahlers verläuft, ist dies anhand einer einzigen Röntgenaufnahme möglich. Die Führungseinrichtung wird dann mittels der Mittel zum Positionieren der Führungseinrichtung in eine solche Position gebracht, daß die Gerade, auf der der Einstichkanal liegt, durch den ermittelten Einstichpunkt verläuft. Da die Mittel zum Positionieren der Führungseinrichtung selbsttätig derart wirken, daß die Gerade, auf der der Einstichkanal liegt, für beliebige Positionen der Führungseinrichtung durch den Fokus des Röntgenstrahlers verläuft, ist sichergestellt, daß auch das diagnostisch relevante Objekt auf der Geraden liegt. Wird nun die Biopsienadel zunächst in den Kanal der Führungseinrichtung eingeführt, dann in das Untersuchungsobjekt eingestochen und anschließend in die erforderliche Tiefe vorgetrieben, ist es ausgeschlossen, daß die Biopsienadel an dem diagnostisch relevanten Objekt vorbeigeführt wird.

Für den Fall, daß der Strahlungsempfänger dem Röntgenbild entsprechende elektrische Signale abgibt, die zur Darstellung des Röntgenbildes einem Fernsehmonitor zugeführt sind, ist nach einer Variante der Erfindung vorgesehen, daß die Mittel zur Bestimmung des Einstichpunktes Mittel zum Einblenden einer Marke, z.B. eines Fadenkreuzes, in das auf dem Fernsehmonitor dargestellte Röntgenbild und Mittel zum Bewegen der Marke in dem Röntgenbild aufweisen und daß die Mittel zum Positionieren der Führungseinrichtung derart ausgebildet sind, daß diese selbsttätig motorisch in eine solche Lage relativ zu dem Untersuchungsobjekt verstellbar ist, daß die Gerade, auf der der Einstichkanal liegt, durch die fiktive Position der Marke auf dem Strahlungsempfänger verläuft. Es ist dann lediglich erforderlich, die Marke auf dem Bildschirm des Fernsehmonitors auf das diagnostisch relevante Objekt zu verstellen und die Mittel zum Positionieren der Führungseinrichtung zu betätigen, um die Führungseinrichtung in die erforderliche Position zu bringen. Unmittelbar im Anschluß daran kann die Punktion oder Markierung des diagnostisch relevanten Objektes erfolgen.

Für den Fall, daß keine Möglichkeit besteht, das Röntgenbild auf einem Fernsehmonitor darzustellen, weisen die Mittel zur Bestimmung des Einstichpunktes zwei in der Strahlenpyramide befindliche, einen Winkel einschließende, auf dem Röntgenbild abbildbare Maßstäbe auf. Die Mittel zum Positionieren der Führungseinrichtung weisen dann zwei gemeinsam mit dieser verstellbare Markierungen auf, von denen jede mit einem der Maßstäbe zusammenwirkt, wobei jede Markierung relativ zu der Geraden, auf der der Einstichkanal liegt, derart angeordnet ist, daß dessen Längsachse in einer Ebene verläuft, die den entsprechenden Maßstab in jenem Punkt schneidet, auf den die Markierung ausgerichtet ist, und die parallel zu dem jeweils anderen Maßstab verläuft. Um eine Biopsie vorzunehmen, wird dann derart vorgegangen, daß zunächst ein Röntgenbild des Untersuchungsobjektes angefertigt wird, auf dem die beiden Maßstäbe sichtbar sind. Anschließend werden die Koordinaten eines diagnostisch relevanten Objektes anhand der Maßstäbe bestimmt und die Führungseinrichtung unter Zuhilfenahme der mit den Maßstäben zusammenwirkenden Markierungen in eine solche Lage gebracht, daß die Markierungen auf die den Koordinaten des diagnostisch relevanten Objektes entsprechenden Punkte der Maßstäbe gerichtet sind. Da die Markierungen relativ zu dem Kanal jeweils derart angeordnet sind, daß dessen Längsachse in einer Ebene verläuft, die den entsprechenden Maßstab in jenem Punkt schneidet, auf den die Markierung ausgerichtet ist, ist gewährleistet, daß die Gerade, auf der der Einstichkanal liegt, durch das diagnostisch relevante Objekt verläuft. Eine erfindungsgemäße Biopsieeinrichtung, bei der die Maßstäbe im Randbereich der Strahlenpyramide angeordnet sind, kann folgende Merkmale aufweisen:
a) die Mittel zum Positionieren der Führungseinrichtung weisen zwei in unterschiedlichen Ebenen angeordnete Lineale auf, von denen jedes einem der Maßstäbe zugeordnet ist, im rechten Winkel zu diesem verläuft und längs desselben verschiebbar geführt ist,
b) die Lineale weisen eine solche Länge auf, daß sie sich in jeder Stellung durch die gesamte Strahlenpyramide hindurch erstrecken,
c) die mit den Maßstäben zusammenwirkenden Markierungen sind jeweils an dem entsprechenden Lineal angebracht und weisen einen Abstand von dessen Längsachse auf,
d) die Lineale sind jeweils um eine parallel zu ihrer Längsachse verlaufende Achse schwenkbar, wobei jeweils das Schwenken der Lineale beim Verschieben derselben längs des entsprechenden Maßstabs mittels eines zwangsläufigen Mechanismus derart erfolgt, daß der Fokus des Röntgenstrahlers, die Markierung und die Achse des jeweiligen Lineals stets in einer gemeinsamen Ebene liegen, in der auch die Gerade verläuft, auf der der Einstichkanal liegt,
e) die Lineale weisen jeweils eine Anlagefläche für das Führungselement auf, die parallel zu der entsprechenden gemeinsamen Ebene verläuft und sich im wesentlichen über die gesamte Länge des entsprechenden Lineals erstreckt,
f) die Führungseinrichtung ist durch ein Führungselement von quaderförmiger Gestalt gebildet, das in den durch die Anlageflächen gebildeten Winkel einsetzbar ist,
g) die Lineale sind jeweils in eine Parkstellung außerhalb der Strahlenpyramide verfahrbar.

Die zwangsläufigen Mechanismen zum Schwenken der Lineale können dabei jeweils eine feststehende Kulisse aufweisen, mit der das entsprechende Lineal mit einem im Abstand von der jeweiligen Achse angebrachten Vorsprung in Eingriff steht.

Um zu verhindern, daß während der Vornahme einer Punktion oder Markierung eines diagnostisch relevanten Objektes Verschiebungen der Führungseinrichtung auftreten, kann vorgesehen sein, daß die Lineale gegen Verschiebungen längs der Maßstäbe arretierbar sind.

Um die Führungseinrichtung möglichst genau positionieren zu können, ist es vorteilhaft, wenn die Markierung jedes Lineals relativ zu dem entsprechenden Maßstab derart angeordnet ist, daß Parallaxenfehler vermieden sind.

Um Biopsienadeln unterschiedlicher Durchmesser verwenden zu können, ist es zweckmäßig, wenn die Führungseinrichtung nach einer Ausführung der Erfindung austauschbar ist. Dies ist auf einfache Weise möglich, wenn die Führungseinrichtung magnetisch gehaltert ist.

Im Hinblick auf die einzuhaltende Hygiene ist es außerdem von Vorteil, wenn die Führungseinrichtung durch ein Führungselement gebildet ist, das aus zwei Teilen besteht, deren Trennfläche einen Kanal für die Biopsienadel enthält, dessen Durchmesser wenigstens im wesentlichen dem der Biopsienadel entspricht, da dann eine einfache Reinigung des Führungselementes und des Kanales gewährleistet ist. Außerdem bietet diese Maßnahme den Vorteil, daß die Biopsienadel in dem Untersuchungsobjekt verbleiben kann, wenn dieses von der Biopsieeinrichtung getrennt wird. Dies ist insbesondere dann von Vorteil, wenn das Untersuchungsobjekt zur Anfertigung einer Kontrollaufnahme umgelagert werden soll oder die Biopsienadel für einen unmittelbar folgenden operativen Eingriff als Markierung in dem Untersuchungsobjekt verbleiben soll.

In den beigefügten Zeichungen sind Ausführungsbeispiele der Erfindung dargestellt. Es zeigen:
- Fig. 1: in schematischer perspektivischer Darstellung ein Röntgenuntersuchungsgerät mit einer erfindungsgemäßen Biopsieeinrichtung,
- Fig. 2: in teilweise schematischer Darstellung eine Seitenansicht eines Röntgenuntersuchungsgerätes mit einer erfindungsgemäßen Biopsieeinrichtung,
- Fig. 3: eine weitere Seitenansicht des Röntgenuntersuchungsgerätes nach Fig. 2, und
- Fig. 4: eine Aufsicht auf die Biopsieeinrichtung des Röntgenuntersuchungsgerätes nach Fig. 2 und 3.

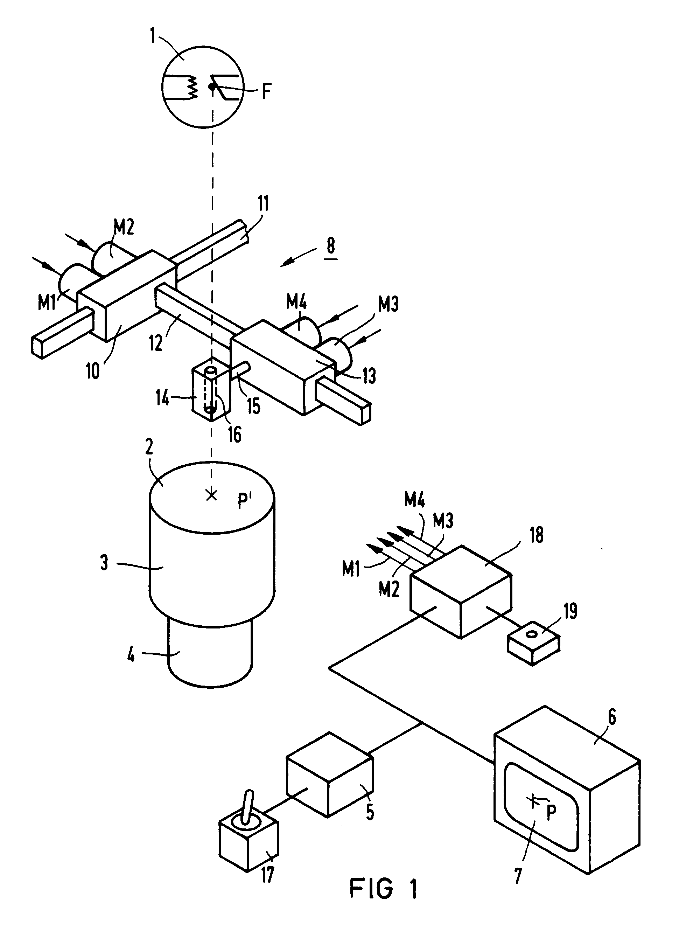

Das in Fig. 1 dargestellte Röntgenuntersuchungsgerät weist eine Röntgenröhre 1 mit einem Fokus F auf, von dem eine Strahlenpyramide ausgeht. Die Strahlenpyramide trifft, nachdem sie ein in Fig. 1 nicht dargestelltes Untersuchungsobjekt durchdrungen hat, auf den Eingangsleuchtschirm 2 eines Röntgenbildverstärkers 3 auf, dem eine Fernsehkamera 4 nachgeschaltet ist, die das Bild des Ausgangsleuchtschirms des Röntgenbildverstärkers 3 aufnimmt. Die von der Fernsehkamera 4 abgegebenen Signale gelangen über eine Bildverarbeitungseinrichtung 5 zu einem Fernsehmonitor 6, auf dessen Bildschirm 7 das Röntgenbild des Untersuchungsobjektes zur Darstellung kommt. Zwischen dem Röntgenstrahler 1 und dem in Fig. 1 nicht dargestellten Untersuchungsobjekt ist eine insgesamt mit 8 bezeichnete Biopsieeinrichtung in einem festen Abstand von dem Eingangsleuchtschirm 2 angeordnet, die einen ersten Wagen 10 aufweist, der auf einer rechteckigen Führungsstange 11 mittels eines Motors M1 verfahrbar ist. An dem ersten Wagen 10 ist eine weitere rechteckige Führungsstange 12 angebracht, die mittels des Motors M2 um ihre Längsachse schwenkbar ist, derart, daß ein auf der Führungsstange 12 mittels des Motors M3 verfahrbarer zweiter Wagen 13 der Schwenkbewegung der Führungsstange 12 folgt. An dem zweiten Wagen 13 ist als Führungseinrichtung für die Biopsienadel ein Führungselement 14 um eine Achse 15 mittels des Motors M4 schwenkbar angebracht. Das Führungselement 14 weist einen Kanal 16 auf, durch den mit der nicht dargestellten Biopsienadel in das unterhalb des Führungselementes 14 befindliche Untersuchungsobjekt eingestochen werden kann. Zur Bestimmung des Einstichpunktes für die Biopsienadel ist ein Joystick 17 vorgesehen, der an die Bildverarbeitungseinrichtung 5 angeschlossen ist, die abhängig von der Stellung des Joysticks 17 eine Marke P in das auf dem Bildschirm 7 dargestellte Röntgenbild einblendet, die mittels des Joysticks 17 beliebig auf dem Bildschirm bewegbar und somit auf ein in dem Röntgenbild sichtbares diagnostisch relevantes Objekt einstellbar ist. Von der Bildverarbeitungseinrichtung 5 werden der Position der Marke P entsprechende Signale an eine Motorsteuerungseinrichtung 18 gegeben. Sobald ein mit dieser verbundener Taster 19 gedrückt wird, betätigt die Motorsteuerungseinrichtung 18 die Motore M1 bis M4 derart, daß das Führungselement 14 in eine solche Position relativ zu dem Untersuchungsobjekt verfahren wird, daß die Längsachse seines Kanales 16 durch den zu dem diagnostisch relevanten Objekt gehörigen Einstichpunkt verläuft, der aufgrund des Umstandes, daß das Röntgenbild durch Zentralprojektion entsteht, auf einer Geraden liegt, die durch das diagnostisch relevante Objekt und den Fokus F des Röntgenstrahlers 1 verläuft Die Motorsteuerungseinrichtung 18 ist so ausgebildet, daß sie die Motore M1 bis M4 selbsttätig in der Weise betätigt, daß die in Fig. 1 strichliert eingetragene Längsachse des Kanales 16 des Führungselementes 14 durch den Fokus F der Röntgenröhre 1 und die fiktive Position P′ der Marke auf dem Eingangsleuchtschirm 2 des Röntgenbildverstärkers 3 verläuft. Es ist somit sichergestellt, daß der Einstichkanal der Biopsienadel, die durch den Kanal 16 des Führungselementes 14 in das Untersuchungsobjekt eingeführt wird zu dem diagnostisch relevanten Objekt innerhalb des Untersuchungsobjektes führt.

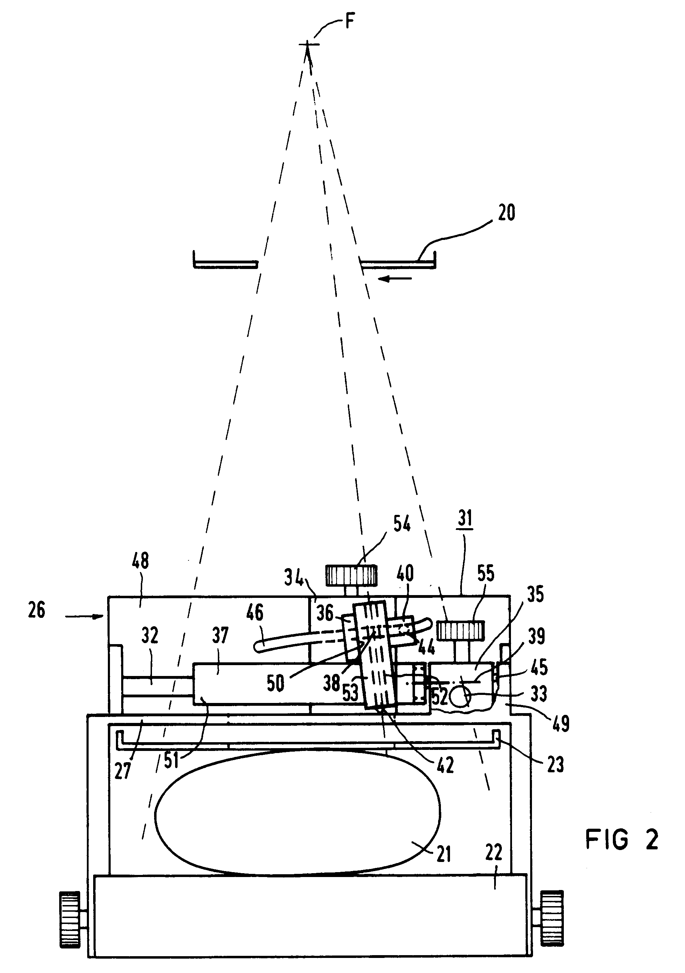

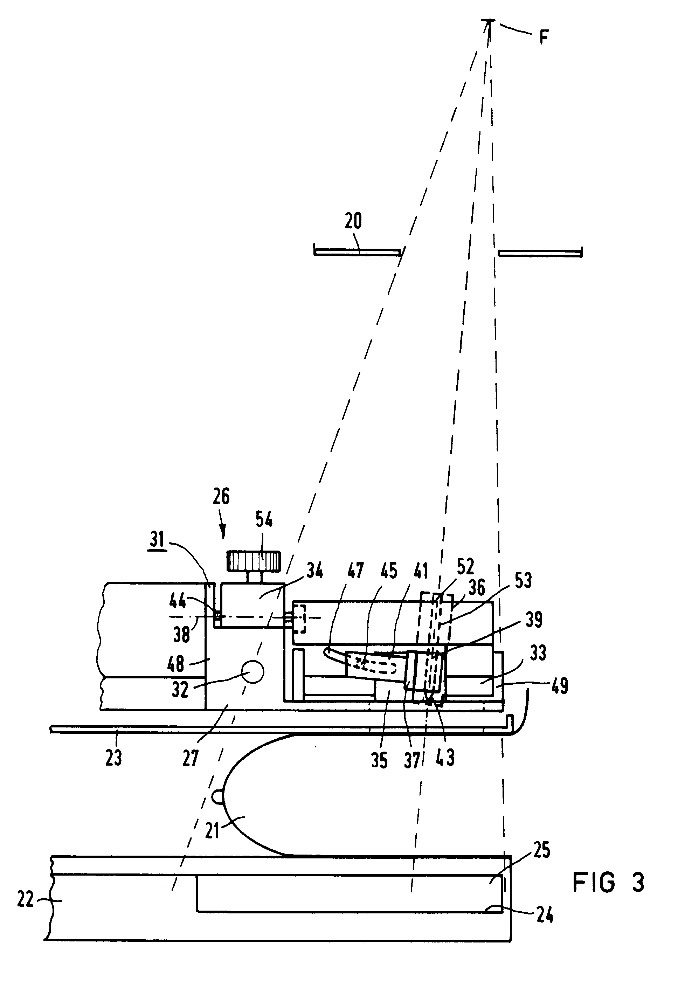

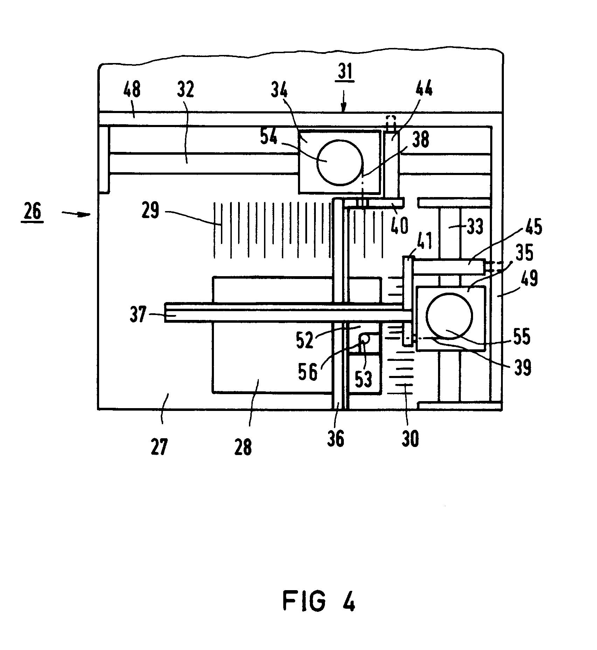

In den Figuren 2 bis 4 ist ein Röntgenuntersuchungsgerät mit einer anderen Ausführungsform der erfindungsgemäßen Biopsieeinrichtung dargestellt. Im einzelnen weist dieses Röntgenuntersuchungsgerät einen Röntgenstrahler auf, von dem nur der Fokus F dargestellt ist. Das von diesem ausgehende Röntgenstrahlenbündel wird mittels einer Primärstrahlenblende 20 derart eingeblendet, daß eine strichliert angedeutete Strahlenpyramide rechteckigen Querschnitts entsteht, in deren Strahlengang sich das Untersuchungsobjekt 21, z.B. eine weibliche Brust, befindet. Das Untersuchungsobjekt 21 liegt auf einer Auflageplatte 22 auf und ist mittels einer relativ zu dieser höhenverstellbaren, eine Öffnung aufweisenden Kompressionsplatte 23 aus strahlendurchlässigem Material komprimierbar. Die Auflageplatte 22 besitzt einen Schacht 24 in den eine Röntgenfilmkassette 25 einführbar ist.

Oberhalb der Kompressionsplatte 23 ist die insgesamt mit 26 bezeichnete Biopsieeinrichtung in einem festen Abstand zu dem Fokus F des Röntgenstrahlers angebracht. Die Biopsieeinrichtung 26 weist eine zu der Auflageplatte 22 parallele Grundplatte 27 aus einem strahlendurchlässigen Werkstoff auf, die mit einem rechteckigen Ausschnitt 28 versehen ist, der der Öffnung der Kompressionsplatte 23 angepaßt ist und durch den das Untersuchungsobjekt 21 zugänglich ist.

Parallel zu zwei einander benachbarten Kanten des Ausschnittes 28 ist jeweils ein Maßstab 29 bzw. 30 vorgesehen. Deren Teilstriche bestehen jeweils aus einem strahlenundurchlässigen Material, z.B. Blei, und sind, da sie sich in der von dem Fokus F ausgehenden Strahlenpyramide befinden, gemeinsam mit dem Untersuchungsobjekt 21 auf einem Röntgenbild abbildbar. Die Grundplatte 27 weist einen Rand 31 auf, an dem zwei parallel zu den Maßstäben 29 bzw. 30 und der Grundplatte 27 verlaufende Führungsstangen 32 bzw. 33 angebracht sind, die in unterschiedlichen Ebenen angeordnet sind. Auf diesen ist jeweils ein Führungsblock 34 bzw. 35 längsverschiebbar, der ein Lineal 36 bzw. 37 rechteckigen Querschnitts trägt, dessen Längsachse rechtwinklig zu der entsprechenden Führungsstange 32 bzw. 33 und parallel zu der Grundplatte 27 verläuft. Die Lineale 36 und 37, die ebenfalls in unterschiedlichen Ebenen angeordnet sind und eine solche Länge aufweisen, daß sie sich in jeder Stellung durch die gesamte Strahlenpyramide hindurch erstrecken, besitzen jeweils an ihrem der entsprechenden Führungsstange 32 bzw. 33 benachbarten Ende eine rechtwinklige Abwinkelung 40 bzw. 41, an der eine mit dem jeweiligen Maßstab 29 bzw. 30 zusammenwirkende Markierung 42 bzw. 43 angebracht ist, die einen Abstand von der Längsachse des Lineals 36 bzw. 37 aufweist. Die Lineale 36 und 37 sind um parallel zu ihren Längsachsen verlaufende Achsen 38 bzw. 39 schwenkbar an den Führungsblöcken 34 bzw. 35 angebracht, wobei das Schwenken der Lineale 36 und 37 beim Verschieben derselben längs der Führungsstangen 32 und 33 jeweils zwangsläufig derart erfolgt, daß im Falle jedes Lineals 36 bzw. 37 der Fokus F des Röntgenstrahlers, die Markierung 42 bzw. 43 und die Achse 38 bzw. 39 in einer gemeinsamen Ebene liegen. Zu diesem Zweck ist an den Abwinkelungen 40 und 41 der Lineale 36 und 37 jeweils ein Vorsprung 44 bzw. 45 vorgesehen, der in eine relativ zu den Linealen 36 bzw. 37 feststehende Nut 46 bzw. 47 eingreift, die in dem parallel zu der jeweiligen Führungsstange 32 bzw. 33 verlaufenden Abschnitt 48 bzw. 49 des Randes 31 der Grundplatte 27 angebracht ist. Das Maß, um das im Falle des beschriebenen Ausführungsbeispiels die Nuten 46 und 47 von einem geradlinigen Verlauf parallel zu den Führungsstangen 32 und 33 abweichen, entspricht dem Cosinus des zwischen der jeweiligen gemeinsamen Ebene und der entsprechenden Führungsstange 32 bzw. 33 eingeschlossenen Winkels multipliziert mit dem Abstand zwischen der jeweiligen Achse 38 bzw. 39 und dem zugehörigen Vorsprung 44 bzw. 45.

Die Lineale 36 und 37 weisen jeweils eine Anlagefläche 50 bzw. 51 für ein quaderförmiges Führungselement 52 auf, das als Führungseinrichtung für eine Biopsienadel dient und das in dem durch die Anlageflächen 50 und 51 der Lineale 36 und 37 gebildeten Winkel magnetisch gehaltert ist. Die Anlageflächen 50 und 51 der Lineale 36 und 37 verlaufen jeweils parallel zu der gemeinsamen Ebene, in der sich der Fokus F des Röntgenstrahlers, die jeweilige Markierung 42 bzw. 43 und die jeweilige Achse 38 bzw. 39 befinden. In dem Führungselement 52 ist ein Kanal 53 für die nicht dargestellte Biopseinadel vorgesehen, wobei jeweils ein Führungselement eingesetzt wird, dessen Kanal 53 in seinem Durchmesser der verwendeten Biopsienadel im wesentlichen entspricht. Der Kanal 53 ist in dem Führungselement 52 derart angeordnet, daß er parallel zu den Anlageflächen 50 und 51 verläuft, und zwar jeweils in einem Abstand von diesen, der dem Abstand zwischen den Anlageflächen 50 bzw. 51 und den zugehörigen oben erwähnten gemeinsamen Ebenen entspricht.

Durch diese Maßnahmen ist sichergestellt, daß in jeder Stellung der Lineale 36, 37 der Verlauf der Längsachse des Kanals 53 der Schnittgeraden zweier Ebenen entspricht, die jeweils parallel zu einem der Maßstäbe 29 bzw. 30 und durch die mit dem jeweils anderen Maßstab 30 bzw. 29 zusammenwirkende Markierung 43 bzw. 42 sowie den Fokus F des Röntgenstrahlers verlaufen. Dies bedeutet, daß in jeder Stellung der Lineale 36, 37 die Längsachse des Kanales 53 durch den Fokus F des Röntgenstrahlers verläuft. Soll also ein auf einem Röntgenbild sichtbares, innerhalb des Untersuchungsobjektes 21 befindliches diagnostisch relevantes Objekt punktiert werden, genügt es, anhand der auf dem Röntgenbild abgebildeten Maßstäbe 29 und 30 die Koordinaten dieses Objektes zu bestimmen und die Lineale 36 und 37 durch Verstellen der Führungsblöcke 34 und 35 in eine solche Position zu bringen, daß die Markierungen 42 und 43 auf die den Koordinaten des diagnostisch relevanten Objektes entsprechenden Teilstriche der Maßstäbe 29 und 30 ausgerichtet sind. Man erhält dann einen Einstichpunkt für die Biopsienadel, der aufgrund des Umstandes, daß das Röntgenbild durch Zentralprojektion entstehnt, auf einer Geraden liegt, die durch den Fokus F des Röntgenstrahlers und das diagnostisch relevante Objekt verläuft. Da außerdem die Längsachse des Kanales 53 des Führungselementes 52 durch den Fokus F des Röntgenstrahlers verläuft, wenn das Führungselement 52 in den durch die Anlageflächen 50 und 51 der Lineale 36 und 37 gebildeten Winkel eingesetzt ist, ist selbsttätig sichergestellt, daß der Einstichkanal einer durch den Kanal 53 des Führungselementes 52 in das Untersuchungsobjekt 21 eingeführten Biopsienadel zu dem diagnostisch relevanten Objekt führt. Bei der Durchführung der Punktion ist es zweckmäßig, die Führungsblöcke 34 und 35 vor der Punktion durch Betätigen der auf die Führungsstangen 32 und 33 wirkenden Klemmschrauben 54 und 55 zu arretieren.

Zur Anfertigung von Röntgenbildern werden die Lineale 36 und 37 übrigens jeweils in eine Parkposition verfahren, in der sie sich außerhalb der Strahlenpyramide befinden.

Wie in den Fig. 2 bis 4 angedeutet ist, besteht das Führungselement 52 aus zwei Teilen, deren Trennfuge den Kanal 53 enthält.

## Patentansprüche

1. Biopsieeinrichtung für ein Röntgenuntersuchungsgerät mit einem Röntgenstrahler (1) mit einem Fokus (F), von dem eine Strahlenpyramide ausgeht, und einem diesem gegenüberliegend angeordneten Strahlungsempfänger (3; 25) zur Erzeugung eines Röntgenbildes eines zwischen beiden befindlichen Untersuchungsobjektes (21), welche Biopsieeinrichtung Mittel (5, 6, 17; 29, 30) zur Bestimmung eines zu einem auf dem Röntgenbild abgebildeten, innerhalb des Untersuchungsobjektes (21) befindlichen diagnostisch relevanten Objekt gehörigen Einstichpunktes für eine Biopsienadel aufweist, der auf einer Geraden liegt, die durch das diagnostisch relevante Objekt und den Fokus (F) des Röntgenstrahlers (1) verläuft, und außerdem eine Führungseinrichtung (14; 52) zum Ausrichten der Biopsienadel auf den jeweiligen Einstichpunkt und Mittel (5, 10 bis 13, 15, 18, 19, M1 bis M4; 32 bis 39, 42 bis 47) zum Positionieren der Führungseinrichtung (14; 52) relativ zu dem Untersuchungsobjekt (21) aufweist, mittels derer die Führungseinrichtung (14; 52) selbsttätig derart positionierbar ist, daß der Einstichkanal der in der Führungseinrichtung (14; 52) aufgenommenen Biopsienadel für beliebige Positionen der Führungseinrichtung (14; 52) relativ zu dem Untersuchungsobjekt auf der Geraden durch den Fokus (F) des Röntgenstrahlers (1) und das diagnostisch relevante Objekt liegt.

2. Biopsieeinrichtung nach Anspruch 1, bei der der Strahlungsempfänger (2, 3, 4) dem Röntgenbild entsprechende elektrische Signale abgibt, die zur Darstellung des Röntgenbildes einem Fernsehmonitor (6, 7) zugeführt sind, **dadurch gekennzeichnet**, daß die Mittel zur Bestimmung des Einstichpunktes Mittel (5) zum Einblenden einer Marke (P) in das auf dem Fernsehmonitor (6, 7) dargestellte Röntgenbild und Mittel (5, 17) zum Bewegen der Marke (P) in dem Röntgenbild aufweisen und daß die Mittel (5, 10 bis 13, 15, 18, 19, M1 bis M4) zum Positionieren der Führungseinrichtung (14) derart ausgebildet sind, daß diese selbsttätig motorisch in eine solche Lage relativ zu dem Untersuchungsobjekt verstellbar ist, daß die Gerade, auf der der Einstichkanal liegt, durch die fiktive Position (P') der Marke (P) auf dem Strahlungsempfänger (2) verläuft.

3. Biopsieeinrichtung nach Anspruch 1, bei der die Mittel zur Bestimmung des Einstichpunktes zwei in der Strahlenpyramide befindliche, einen Winkel einschließende, auf dem Röntgenbild abbildbare Maßstäbe (29, 30) aufweisen, **dadurch gekennzeichnet**, daß die Mittel (32 bis 39, 44 bis 47) zum Positionieren der Führungseinrichtung (52) zwei gemeinsam mit dieser verstellbare Markierungen (42, 43) aufweisen, von denen jede mit einem der Maßstäbe (29, 30) zusammenwirkt, wobei jede Markierung (42, 43) relativ zu der Geraden, auf der der Einstichkanal liegt, derart angeordnet ist, daß die Gerade in einer Ebene verläuft, die den entsprechenden Maßstab (29, 30) in jenem Punkt schneidet, auf den die Markierung (42, 43) ausgerichtet ist, und parallel zu dem jeweils anderem Maßstab (30, 29) verläuft.

4. Biopsieeinrichtung nach Anspruch 3, bei der die Maßstäbe (29, 30) im Randbereich der Strahlenpyramide angeordnet sind, **gekennzeichnet durch** folgende Merkmale:
a) die Mittel zum Positionieren der Führungseinrichtung (52) weisen zwei in unterschiedlichen Ebenen angeordnete Lineale (36, 37) auf, von denen jedes einem der Maßstäbe (29, 30) zugeordnet ist, im rechten Winkel zu diesem verläuft und längs desselben verschiebbar geführt ist,
b) die Lineale (36, 37) weisen eine solche Länge auf, daß sie sich in jeder Stellung durch die gesamte Strahlenpyramide hindurch erstrecken,
c) die mit den Maßstäben (29, 30) zusammenwirkenden Markierungen (42, 43) sind jeweils an dem entsprechenden Lineal (36 bzw. 37) angebracht und weisen einen Abstand von dessen Längsachse auf,
d) die Lineale (36, 37) sind jeweils um eine parallel zu ihrer Längsachse verlaufende Achse (38, 39) schwenkbar, wobei jeweils das Schwenken der Lineale (36, 37) beim Verschieben derselben längs des entsprechenden Maßstabs (29 bzw. 30) mittels eines zwangsläufigen Mechanismus (44, 46 bzw. 45, 47) derart erfolgt, daß der Fokus (F) des Röntgenstrahlers, die Markierung (42 bzw. 43) und die Achse (38 bzw. 39) des jeweiligen Lineals (36 bzw. 37) stets in einer gemeinsamen Ebene liegen, in der auch die Gerade verläuft, auf der der Einstichkanal liegt.
e) die Lineale weisen jeweils eine Anlagefläche (50, 51) für die Führungseinrichtung (52) auf, die parallel zu der entsprechenden gemeinsamen Ebene verläuft und sich im wesentlichen über die gesamte Länge des Lineals (36, 37) erstreckt,
f) die Führungseinrichtung (52) ist durch ein Führungselement von quaderförmiger Gestalt gebildet, das in den durch die Anlageflächen (50, 51) gebildeten Winkel einsetzbar ist,
g) die Lineale (36, 37) sind jeweils in eine Parkstellung ausserhalb der Strahlenpyramide verfahrbar.

5. Biopsieeinrichtung nach Anspruch 4, **dadurch gekennzeichnet**, daß die zwangsläufigen Mechanismen zum Schwenken der Lineale (36, 37) jeweils eine feststehende Kulisse (46, 47) aufweisen, mit der das entsprechende Lineal (36 bzw. 37) mit einem im Abstand von der jeweiligen Achse (38 bzw. 39) angebrachten Vorsprung (44 bzw. 45) in Eingriff steht.

6. Biopsieeinrichtung nach Anspruch 4 oder 5, **dadurch** **gekennzeichnet**, daß die Lineale (36, 37) gegen Verschiebungen längs der entsprechenden Maßstäbe (29, 30) arretierbar sind.

7. Biopsieeinrichtung nach einem der Ansprüche 2 bis 6, **dadurch gekennzeichnet,** daß die Markierungen (42, 43) jeweils relativ zu dem entsprechenden Maßstab (29 bzw. 30) derart angeordnet sind, daß Parallaxenfehler vermieden sind.

8. Biopsieeinrichtung nach einem der Ansprüche 1 bis 7,**dadurch gekennzeichnet,** daß die Führungseinrichtung (14, 52) austauschbar ist.

9. Biopsieeinrichtung nach einem der Ansprüche 4 bis 8, **dadurch gekennzeichnet,** daß die Führungseinrichtung (52) magnetisch gehaltert ist.

10. Biopsieeinrichtung nach einem der Ansprüche 1 bis 9, **dadurch gekennzeichnet**, daß die Führungseinrichtung (14, 52) durch ein Führungselement gebildet ist, das aus zwei Teilen besteht, deren Trennfläche einen Kanal (16, 53) für die Biopsienadel enthält, dessen Durchmesser wenigstens im wesentlichen dem der Biopsienadel entspricht.

## Claims

1. Biopsy device for an X-ray examination apparatus having an X-ray source (1) with a focus (F), from which a pyramid of rays issues, and having a radiation receiver (3; 25) which is arranged lying opposite said focus for the generation of an X-ray image of an examination object (21) located between the two, which biopsy device has means (5, 6, 17; 29, 30) for determining a puncture point for a biopsy needle which point pertains to a diagnostically relevant object, mapped on the X-ray image and located within the examination object (21), and which point lies on a straight line running through the diagnostically relevant object and the focus (F) of the X-ray source (1), and, moreover, a guiding device (14; 52) for the alignment of the biopsy needle with the respective puncture point and means (5, 10 to 13, 15, 18, 19, M1 to M4; 32 to 39, 42 to 47) for positioning the guiding device (14; 52) relative to the examination object (21), by which means the guiding device (14; 52) can be automatically positioned in such a way that the puncture channel of the biopsy needle received in the guiding device (14; 52) for any positions of the guiding device (14; 52) relative to the examination object lies on the straight line through the focus (F) of the X-ray source (1) and the diagnostically relevant object.

2. Biopsy device according to claim 1, in which the radiation receiver (2, 3, 4) emits electrical signals which correspond to the X-ray image and which are supplied to a television monitor (6, 7) for the representation of the X-ray image, characterised in that the means for determining the puncture point have means (5) for mixing a mark (P) into the X-ray image represented on the television monitor (6, 7) and means (5, 17) for moving the mark (P) in the X-ray image and in that the means (5, 10 to 13, 15, 18, 19, M1 to M4) for positioning the guiding device (14) are formed in such a way that the latter can be automatically adjusted by motor into such a position relative to the examination object that the straight line, on which the puncture channel lies, runs through the fictitious position (P') of the mark (P) on the radiation receiver (2).

3. Biopsy device according to claim 1, in which the means for determining the puncture point have two scales (29, 30) which are located in the pyramid of rays, enclose an angle and can be mapped on the X-ray image, characterised in that the means (32 to 39, 44 to 47) for positioning the guiding device (52) have two markings (42, 43) which can be adjusted together with the guiding device and each of which cooperates with one of the scales (29, 30), with each marking (42, 43) being arranged relative to the straight line, on which the puncture channel lies, in such a way that the straight line runs in a plane which intersects the corresponding scale (29, 30) at each point with which the marking (42, 43) is aligned, and runs parallel to the other scale (30, 29) respectively.

4. Biopsy device according to claim 3, in which the scales (29, 30) are arranged in the edge region of the pyramid of rays, characterised by the following features:
a) the means for positioning the guiding device (52) have two rules (36, 37) arranged in different planes, each of which rules is associated with one of the scales (29, 30), runs at a right angle to the latter and is guided in a displaceable manner along the same;
b) the rules (36, 37) are of such a length that in each position they extend through the whole pyramid of rays;
c) the markings (42, 43) cooperating with the scales (29, 30) are provided on the corresponding rule (36 or 37) respectively and are at a distance from the longitudinal axis of the latter;
d) the rules (36, 37) can be swung, in each case, about an axis (38, 39) which runs parallel to their longitudinal axis, with, in each case, the swing of the rules (36, 37) with displacement of the same along the corresponding scale (29 or 30 respectively) being effected by means of a constrained mechanism (44, 46 or 45, 47 respectively) in such a way that the focus (F) of the X-ray source, the marking (42 and 43 respectively) and the axis (38 and 39) of the respective rule (36 and 37 respectively) always lie in a common plane in which there also runs the straight line on which the puncture channel lies;
e) the rules each have a seating surface (50, 51) for the guiding device (52) which runs parallel to the corresponding common plane and extends substantially over the whole length of the rule (36, 37);
f) the guiding device (52) is formed by a guiding element of parallelepiped block-shaped form which can be inserted into the angle formed by the seating surfaces (50, 51);
g) the rules (36, 37) can each be moved into a parking position outside the pyramid of rays.

5. Biopsy device according to claim 4, characterised in that the constrained mechanisms for swinging the rules (36, 37) each have a fixed groove (46, 47) with which the corresponding rule (36 or 37 respectively) is engaged with a projection (44 or 45 respectively) provided at a distance from the respective axis (38 or 39).

6. Biopsy device according to claim 4 or 5, characterised in that the rules (36, 37) can be arrested to prevent their displacement along the corresponding scales (29, 30).

7. Biopsy device according to one of the claims 2 to 6, characterised in that the markings (42, 43) are each arranged relative to the corresponding scale (29 or 30 respectively) in such a way that parallax errors are avoided.

8. Biopsy device according to one of the claims 1 to 7, characterised in that the guiding device (14, 52) can be exchanged.

9. Biopsy device according to one of the claims 4 to 8, characterised in that the guiding device (52) is supported magnetically.

10. Biopsy device according to one of the claims 1 to 9, characterised in that the guiding device (14, 52) is formed by a guiding element which consists of two parts, the interface of which contains a channel (16, 53) for the biopsy needle, the diameter of which at least substantially corresponds to that of the biopsy needle.

## Revendications

1. Dispositif de biopsie pour un appareil de radiodiagnostic comportant un émetteur radiologique (1) possédant un foyer (F), d'où part un rayonnement pyramidal, et un récepteur de rayonnement (3;25) disposé en vis-à-vis de cet émetteur et servant à produire une radiographie d'un objet d'examen (21) situé entre ces deux unités, lequel dispositif de biopsie Comporte des moyens (5,6,7;29,30) pour déterminer un point de perforation pour une aiguille de biopsie, qui est associé à un objet important du point de vue du diagnostic, dont l'image est formée sur la radiographie et qui se trouve à l'intérieur de l'objet d'examen (21), et qui est situé sur une droite passant par l'objet important du point de vue diagnostic et le foyer (F) de l'émetteur radiologique (1), et en outre un dispositif de guidage (14; 52) servant à aligner l'aiguille de biopsie sur le point de perforation respectif, et des moyens (5,10 à 13,15, 18,19,M1 à M4; 32 à 29; 42 à 47) pour positionner le dispositif de guidage (14;52) par rapport à l'objet d'examen (21) et au moyen duquel le dispositif de guidage (14;52) peut être positionné automatiquement de telle sorte que le canal de perforation de l'aiguille de biopsie logée dans le dispositif de guidage (14;52) est situé, pour n'importe quelle position du dispositif de guidage (14;52) par rapport à l'objet d'examen, sur la droite passant par le foyer (F) de l'émetteur radiologique (1) et l'objet important du point de vue diagnostic.

2. Dispositif de biopsie suivant la revendication 1, dans lequel le récepteur de rayonnement (2,3,4) délivre des signaux électriques qui correspondent à la radiographie et qui sont envoyés, pour la représentation de la radiographie, à un moniteur de télévision (6,7), caractérisé par le fait que les moyens pour déterminer le point de perforation comprennent des moyens (5) pour insérer une marque (P) dans la radiographie représentée sur le moniteur de télévision (6,7), et des moyens (5,17) pour déplacer la marque (P) dans la radiographie, et que les moyens (5,10 à 13, 15, 18, 19, M1 à M4) sont agencés pour le positionnement du dispositif de guidage (14) de sorte que ce dernier peut être amené automatiquement par un moteur, dans une position telle par rapport à un objet d'examen, que la droite, sur laquelle est situé le canal de perforation, passe par la position fictive (P') de la marque (P) dans le récepteur de rayonnement (2).

3. Dispositif de biopsie suivant la revendication 1, dans lequel les moyens pour déterminer le point de perforation possèdent deux échelles de mesure (29,30), qui sont situées dans le rayonnement pyramidal et font entre elles un angle et dont l'image peut être formée sur la radiographie, caractérisé par le fait que les moyens (32 à 39, 44 à 47) utilisés pour le positionnement du dispositif de guidage (52) possèdent deux marques (42,43), qui peuvent être déplacées conjointement avec ce dispositif et dont chacune coopère avec l'une des échelles de mesure (29,30), chaque marque (42,43) étant disposée par rapport à la droite, sur laquelle est situé le canal de perforation, de telle sorte que la droite se situe dans un plan qui recoupe l'échelle de mesure correspondante (29,30) au point, sur lequel la marque (42,43) est alignée, et est parallèle à l'autre échelle de mesure respective (30,29).

4. Dispositif de biopsie suivant la revendication 3, dans lequel les échelles de mesure (29,30) sont disposées dans la zone marginale du rayonnement pyramidal, remarquable par les caractéristiques suivantes :
a) les moyens de positionnement du dispositif de guidage (52) possèdent deux règles (36,37) disposées dans des plans différents et dont chacune est associée à l'une des échelles de mesure (29,30), est perpendiculaire à cette échelle et est guidée de manière à être déplaçable le long de cette échelle,
b) les règles (36,37) possèdent une longueur telle que dans n'importe quelle position, elles traversent ledit rayonnement pyramidal;
c) les marques (42,43), qui coopèrent avec les échelles de mesure (29,30), sont disposées respectivement sur la règle correspondante (36 ou 37) et sont situées à une certaine distance de l'axe longitudinal de cette règle,
d) les règles (36,37) peuvent pivoter respectivement autour d'un axe (38,39) parallèle à leur axe longitudinal, le pivotement des règles (36,37) étant réalisé au moyen d'un mécanisme à commande forcée (44,46 ou 45,47) lors du déplacement des règles le long des échelles correspondantes (29,30), de telle sorte que le foyer (F) de l'émetteur radiologique, la marque (42 ou 43) et l'axe (38,39) de la règle respective (36 ou 37) sont situés en permanence dans un plan commun, dans lequel s'étend également la droite sur laquelle est situé le canal de perforation,
e) les règles possèdent chacune une surface d'application (50,51) pour le dispositif de guidage (52), qui est parallèle au plan commun correspondant et s'étend essentiellement sur toute la longueur de la règle (36,37),
f) le dispositif de guidage (52) est formé par un élément de guidage possédant une forme parallélépipédique et qui peut être inséré dans l'angle formé par les surfaces d'application (50,51),
g) les règles (36,37) peuvent être amenées respectivement dans une position de rangement à l'extérieur du rayonnement pyramidal.

5. Dispositif de biopsie suivant la revendication 4, caractérisé par le fait que les mécanismes à commande forcée comportent chacun, pour le pivotement des règles (36,37), une coulisse fixe (46,47), au moyen de laquelle la règle correspondante (36 ou 37) coagit avec une partie saillante (44 ou 45) montée à distance de l'axe respectif (38 ou 39).

6. Dispositif de biopsie suivant la revendication 4 ou 5, caractérisé par le fait que les règles (36,37) peuvent être bloquées contre tout déplacement le long des échelles de mesure correspondantes (29,30).

7. Dispositif de biopsie suivant l'une des revendications 2 à 6, caractérisé par le fait que les marques (42,43) sont disposées respectivement par rapport à l'échelle de mesure correspondante (29 ou 30) de manière à éviter des erreurs de parallaxe.

8. Dispositif de biopsie suivant l'une des revendications 1 à 7, caractérisé par le fait que le dispositif de guidage (14,52) est remplaçable.

9. Dispositif de biopsie suivant l'une des revendications 4 à 8, caractérisé par le fait que le dispositif de guidage (52) est retenu magnétiquement.

10. Dispositif de biopsie suivant l'une des revendications 1 à 9, caractérisé par le fait que le dispositif de guidage (14,52) est formé par un élément de guidage, qui est constitué de deux éléments, dont la surface de séparation contient un canal (16,53) pour l'aiguille de biopsie, dont le diamètre correspond au moins sensiblement à celui de l'aiguille de biopsie.
